# EUROPEAN PATENT APPLICATION

(11) **EP 1 559 390 A2**
(43) Date of publication of application: **03.08.2005**
(21) Application number: 05001857.1
(22) Date of filing: 28.01.2005
(51) Int. Cl.: A61G 15/14

(54) **Tray device for dental unit**

(30) Priority: 30.01.2004 IT BO20040040
(71) Applicant: Vitali S.r.l., 40013 Castel Maggiore (Bologna) (IT)
(72) Inventor: Vitali, Marco, 40057 Cranarolo Emilia BO (IT); Plazzo, Stefano, 44042 Cento FE (IT)
(74) Representative: Negrini, Elena

(57) **Abstract**

Tray device for dental unit with integrated keyboard for data and command entry into a computer. Device carried out integrating a support mean (2) made of rigid molded polyurethane, whose upper face (3) is provided of an appropriate sealed seat IP65 (4) per a keyboard means (5), for data and commands entry into a computer.

The tray is provided with a raised peripheral edge (6) for containing the dental care tool and objects. In the central portion of the peripheral edge (6) is carried out an opening to facilitate the disinfections operations.

The external upper surface of the keyboard means (5) is coplanar to the upper face (3) to form with this latter a support plane of the device (1).

## Description

The present invention concerns the technical field of the dental unit and particularly refers to a tray device, destined to be fixed to the structure of a dental unit.

There are many known different types of dental unit provided with plates or trays fit for supporting tools and objects of treatment and medication, for instance cutters and drills, lancet, buffer, etc. and connected to the support structure of unit by means of articulated arms that allow the desiderate positioning of it in the work area.

In some cases, such unit also introduces small alphanumeric keyboard for the unit control, for instance to manage the driving of the tools and/or to modify the configuration of the chair and/or the intensity of the light of the working lamp. Such keyboards, that generally are fixed to a portion of the chair or they are directly integrated to the support of the utensils, they have the drawback to be too small to be integrated to the support, not allowing therefore to provide operations and more complex controls, for instance, the connection with a calculator or personal computer online or associated to the unit, to extract from the database information on the patient or to check, in more precise and accurate way, the dental unit.

To avoid this drawback, there are known unit endowed with personal computer keyboards, eventually associated to a monitor linked by means of articulated arms to the structure of the unit.

A drawback of said units consists in the fact to have a plurality of articulated arms, for the keyboard, for the support tablet and for the utensils support, that encumbers the working area, making difficult their contemporaneous employment.

Other drawback consists in that such keyboards can be damaged during the use of the unit, for instance unawares supporting sharp tools or pouring liquids and aggressive and corrosive substances.

Further drawback consists in that such keyboards are not easily disinfectable.

An object of the present invention is to propose a tray device for dental unit also integrating a keyboard for the remote control of a calculator or PC.

Other object is that to propose a tray device with keyboard that is strong and resistant to the bumps and the liquids, and easily disinfectable.

The characteristics of the invention are underlined in the following, with particular reference to the attached drawings, in which:
figure 1 shows a schematic plant view of the tray device of the present invention;
figure 2 shows a side sight view partially sectioned of figure 1 device;
figure 3 shows an axonometric sight view of a variant of the figure 1 device in association with a tool support tablet of a dental unit.

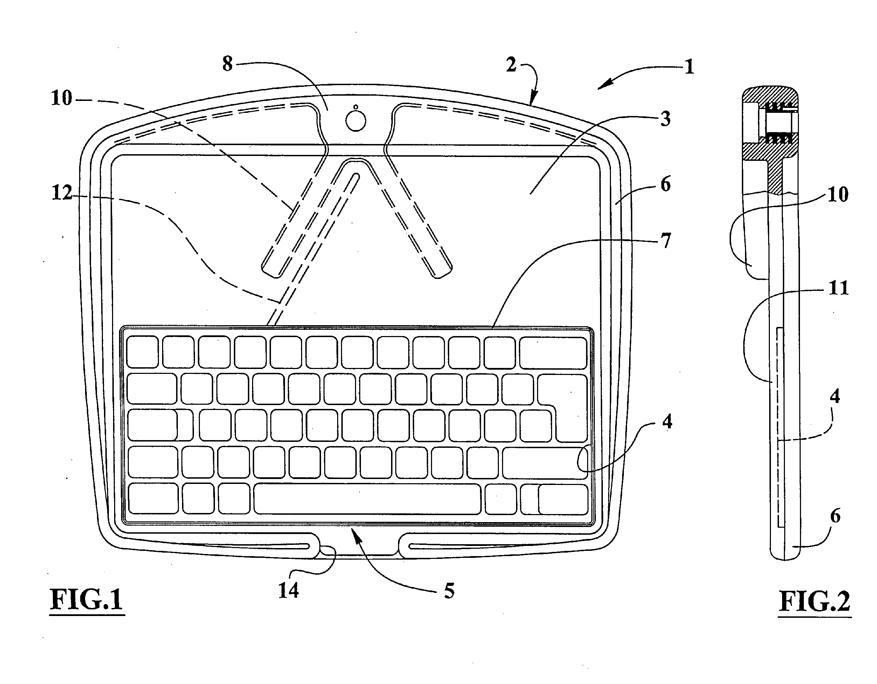

With reference to figures 1 and 2, numeral 1 indicates the tray device for dental unit object of the present invention, including a support means 2 made of rigid formed polyurethane provided, in correspondence of an upper face 3, of a shaped sealed seat 4, fit to house a keyboard means 5 for computer and provided of a raised peripheral edge 6 containing the dental tools.

Sad peripheral edge 6 has an opening 14 for the unload of liquids eventually laying on the device 1.

The external surface of the keyboard means 5 is coplanar and aligned to upper face 3 of the support means 2 to form with this latter an ideal support plane for tools and objects for treatment and medication. The keyboard means 5 can be connected to a PC.

It is important to underline the fact that the invention allows, therefore, to the operator to use at the same time the device 1 both as a tray support an as PC user interface allowing the access to patient database.

The keyboard 5 is of known industrial kind, endowed with completely waterproof and resistant upper surface.

Sealing means 7 are provided preferably comprising one or more Or-ring type gasket, interposed between the shaped seat 4 and the keyboard means 5, for instance, in correspondence of the upper face 3, to ensure the water and dust sealing of the device 1. More precisely, such sealing means 7 allow the device to be fully protected also against the water sprinkling reaching a protection level IP 65 according to international rule IEC 529.

The device is of supported by an arm means 9a of connection mean 9 provided of appropriate stops to avoid internal cable breaking.

The arm means 9a rotates around a brass bush embedded into the rigid polyurethane during the molding step.

The tool seat 13 is fixed to the arm means by means of a iron pivot also provided with stop to avoid the internal cable breaking.

To increase the flexion and torsion rigidity of the device, and to prevent yielding or folding of the support means 2, stiffening means 10 are provided (ribs or relief) carried out on the lower face 11.

Furthermore the device includes an inner duct 12 fit to house connection cables of the keyboard means 5 and fit to conduct them in proximity of the connection portion 8 for their insertion in a special hollow of the connection mean 9.

The support means 2 is made of rigid polyurethane by means of molding process.

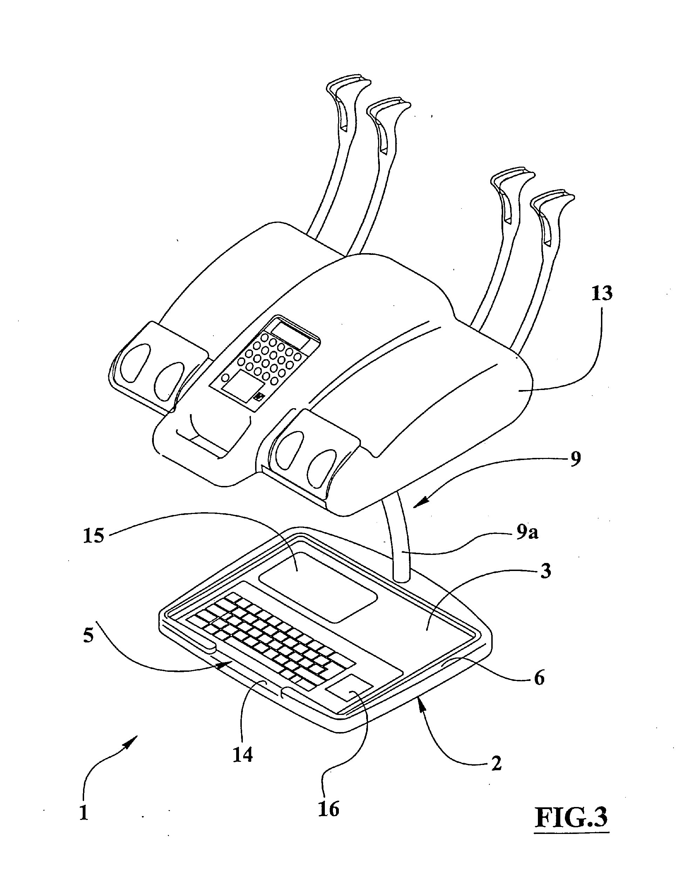

Figure 3 shows a variant of the device 1 in which the support means 2 includes display means 15 integrated in the upper face 3, to show data and essential information concerning the operation of the unit, to the operator.

The keyboard means 5 can further comprise a pointing means 16, a so called pad, in order to facilitate the manual pointing of functions executed by the computer connected to said keyboard means 5.

An advantage of the present invention is to provide a tray device for dental unit that also integrates a keyboard for the remote control of a calculator or PC.

Other advantage is to furnish a strong and resistant device easy to be disinfected.

## Claims

1. Tray device for dental unit **characterized in that** to comprise at least a support mean (2) provided, in correspondence of an upper face (3), of a shaped seat (4) fit to house a keyboard means (5) for data entry and commands and provided with a raised peripheral edge (6); the upper external surface of the keyboard means (5) being coplanar to the upper face (3) to form with this latter a support plane of the device (1).

2. Device according to claim 1 **characterized in that** to comprise sealing means (7) interposed between the shaped seat (4) and the keyboard means (5) and fit to ensure at least the water and dust sealing of the device (1).

3. Device according to claim 1 **characterized in that** to comprise connection mean (9) of the dental unit fixed to a connection portion (8) of the peripheral edge (6).

4. Device according to claim 3 **characterized in that** the connection mean (9) comprises at least a revolving arm fixed to a tool seat (13) of the dental unit.

5. Device according to claim 1 **characterized in that** to comprise stiffening means (10) sited on a lower face (11) of the support mean (2).

6. Device according to claim 1 **characterized in that** to comprise an inside duct (12) fit to house connection cables of the keyboard means (5).

7. Device according to claims 3 and 6 **characterized in that** the connection mean (9) comprises a hollow for the passage of the connection cables of the keyboard means (5).

8. Device according to claim 1 **characterized in that** the support mean (2) is made of rigid polyurethane by means of molding process.

9. Device according to claim 1 **characterized in that** the keyboard means (5) is industrial and waterproof.

10. Device according to claim 1 **characterized in that** the peripheral edge (6) has at least an opening (14) for the unload of liquids eventually laying on the device (1).

11. Device according to claim 1 **characterized in that** said support mean (2) comprises display means (15) integrated in the upper face (3).

12. Device according to claim 1 **characterized in that** said keyboard means (5) is connected to a computer.

13. Device according to claim 1 **characterized in that** the keyboard means (5) comprises at least a pointing means (16).
